# EUROPÄISCHE PATENTSCHRIFT

(11) **EP 1 309 570 B1**
(45) Veröffentlichungstag und Bekanntmachung des Hinweises auf die Patenterteilung: **26.03.2008**
(21) Anmeldenummer: 01971784.2
(22) Anmeldetag: 26.07.2001
(51) Int. Cl.: C07D 251/60

(54) **VERFAHREN ZUR HERSTELLUNG VON MELAMIN**
METHOD FOR THE PRODUCTION OF MELAMINE
PROCEDE DE PREPARATION DE MELAMINE

(30) Priorität: 07.08.2000 AT 13632000
(43) Veröffentlichungstag der Anmeldung: 14.05.2003
(73) Patentinhaber: AMI Agrolinz Melamine International GmbH, 4020 Linz (AT)
(72) Erfinder: COUFAL, Gerhard, A-4060 Leonding (AT); BUCKA, Hartmut, A-4622 Eggendorf 125 (AT); BAIRAMIJAMAL, Faramarz, A-4030 Linz (AT)
(74) Vertreter: Gross, Felix
(86) Internationale Anmeldenummer: PCT/EP2001/008648
(87) Internationale Veröffentlichungsnummer: WO 2002/012206

(56) Entgegenhaltungen:
- WO-A-00/29393
- WO-A-97/20826
- WO-A-99/38852

## Beschreibung

Die Erfindung betrifft die Herstellung von Melamin durch Pyrolyse von Harnstoff mit Hilfe von hintereinandergeschalteten Reaktoren in einem Hochdruckverfahren.

Bei den Hochdruckverfahren zur Herstellung von Melamin wird Harnstoff in einer endothermen Flüssigphasenreaktion zu Melamin umgesetzt. Das flüssige Melamin enthält je nach den Druck- und Temperaturbedingungen im Reaktor zusätzlich unterschiedliche Mengen an gelöstem NH₃ und CO₂ sowie Kondensationsnebenprodukte und nicht umgesetzten Harnstoff. Das so erhaltene unter hohem NH₃-Druck stehende Melamin wird anschließend etwa durch Quenchen mit Wasser oder mit Ammoniak, durch Sublimation mit nachfolgender Desublimation oder durch Entspannen unter bestimmten Bedingungen verfestigt. Als Reaktor wird üblicherweise ein einzelner Apparat vom Rührkesseltyp eingesetzt.
Ein wesentliches Problem bei der Herstellung von Melamin aus Harnstoff besteht darin, daß der eingesetzte Harnstoff nicht vollständig umgesetzt wird und darüber hinaus in den nach dem Stand der Technik üblichen Reaktoren Nebenprodukte gebildet werden, welche anschließend durch kostspielige und komplizierte Aufarbeitungsschritte zu Melamin umgewandelt werden müssen. Es ist beispielsweise aus WO97/20826 bekannt, daß reines Melamin dann erhalten werden kann, wenn das Melamin vor der Verfestigung auf Temperaturen gekühlt wird, die knapp über dem jeweiligen, vom jeweiligen NH₃-Druck abhängigen Schmelzpunkt des Melamins liegen. Die Kühlung des Melamins vor der Verfestigung erfolgt durch Zusatz von NH₃ oder mittels Wärmetauscher.

Es wurde nun unerwarteterweise gefunden, daß die Kühlung des Melamins vor der Verfestigung auch durch Zusatz einer geringen Menge an Harnstoff erfolgen kann, der dabei gleichzeitig in endothermer Reaktion zu Melamin umgesetzt wird. Die Kühlung erfolgt also unter Bildung von weiterem Melamin, analog zur Hauptreaktion der Melaminsynthese.

Gegenstand der Erfindung ist demnach ein Verfahren zur Herstellung von Melamin durch Pyrolyse von Harnstoff in einem Hochdruckverfahren, das dadurch gekennzeichnet ist, daß Harnstoff gegebenenfalls gemeinsam mit NH₃ einem Melaminreaktor zugeführt, dort zu Melamin umgesetzt und das entstehende Offgas am Kopf des Reaktors abgezogen wird, die gebildete Melaminschmelze von oben einem Kühlreaktor zugeführt wird und im Kühlreaktor mit einer solchen Menge Harnstoffs versetzt wird, daß sie auf eine Temperatur abgekühlt wird, die 1 - 50 °C, bevorzugt 1 - 30 °C über dem vom jeweiligen NH₃-Druck abhängigen Schmelzpunkt des Melamins liegt, worauf durch Einleiten von NH₃ im Gegenstrom das gebildete CO₂ ausgetrieben, die Gase am Kopf des Kühlreaktors abgetrennt werden und die Melaminschmelze anschließend in beliebiger Weise aufgearbeitet wird.

Zur Durchführung des erfindungsgemäßen Verfahrens wird Harnstoff, der bevorzugt aus einem Harnstoffwäscher kommt, mit einer Temperatur von etwa 135 - 250°C, von unten in einen Melaminreaktor eingebracht. Gemeinsam mit dem Harnstoff wird gasförmiges NH₃, das sowohl in der aus dem Harnstoffwäscher kommenden Schmelze gelöst ist, als auch zusätzlich eingebracht werden kann, mit einer Temperatur von etwa 150 - 450 °C in den Reaktor von unten eingetragen. Dabei beträgt das Molverhältnis von dem dem Melaminreaktor zugeführten NH₃ zum zugeführten Harnstoff 0-3mol, bevorzugt 0-2mol, besonders bevorzugt etwa 0-1 mol NH₃ / mol Harnstoff. Der Druck im Melaminreaktor liegt in einem Bereich von etwa 50 - 350 bar, bevorzugt von 80 - 250 bar.
Die Temperatur im Melaminreaktor liegt in einem Bereich von etwa 320 - 450°C, bevorzugt von 300 - 400°C, besonders bevorzugt von 330 - 380 °C.

Der in den Melaminreaktor eingebrachte Harnstoff wird in einer endothermen Reaktion zu Melamin, CO₂ und NH₃ umgesetzt. Die erzeugte Melaminschmelze enthält außerdem unterschiedliche Mengen an gelöstem NH3 und CO₂ sowie Kondensationsnebenprodukte und nicht umgesetzten Harnstoff. Aufgrund des Eigendampfdruckes von Melamin enthält das hauptsächlich aus NH₃ und CO₂ bestehende Offgas zusätzlich gasförmiges Melamin.

Als Melaminreaktor kann ein beliebiger Reaktor bzw. mehrere Reaktoren eingesetzt werden, bevorzugt ist ein Tankreaktor, beispielsweise ein Rührreaktor. Die Durchmischung der Reaktionsmasse kann im Rührreaktor entweder durch ein Rührwerk oder mit Hilfe der entstehenden Reaktionsgase erfolgen. Die für die Reaktion benötigte Wärme kann auf verschiedene Weise eingebracht werden. Bevorzugt wird sie über eine in vertikalen Rohren, bevorzugt Doppelmantelrohren im Reaktorinneren eines Rohrbündelreaktors zirkulierende Salzschmelze bereitgestellt. Dabei erfolgt der Zulauf der Salzschmelze üblicherweise über den äußeren Rohrmantel und der Ablauf über den Innenrohrquerschnitt. Die Durchmischung der Reaktionsmasse erfolgt bevorzugt über einen Naturumlauf, der durch die Dichteunterschiede zwischen den entstehenden Reaktionsgasen und der Melaminschmelze erzwungen wird. Harnstoff und NH₃ werden gemeinsam am Boden des Reaktors eingebracht, und zu Melamin und Offgas umgesetzt. Im oberen Reaktorteil trennt sich das Reaktionsgemisch in Offgas und flüssiges Melamin.
Während das Offgas am Kopf des Reaktors kontinuierlich abgezogen wird, fließt der größte Teil der Melaminschmelze durch die Schwerkraft nach unten. Wegen der unterschiedlichen Dichte des Reaktionsgemisches aus Rohmelamin und Offgas einerseits und der vom Offgas befreiten Rohmelaminschmelze findet im Reaktorinneren eine Zirkulation statt. Das gebildete Melamin wird über den im oberen Teil des Reaktors befindlichen Überlauf aus dem Reaktor ausgetragen. Das gebildete Offgas wird einem Offgaswäscher zugeführt, während das Melamin einem Kühlreaktor zugeführt wird, wobei der Eintrag im oberen Teil des Kühlreaktors erfolgt.

Dem Kühlreaktor wird neben der Melaminschmelze, die aus dem Melaminreaktor kommt, soviel Harnstoff zugesetzt, daß dabei die Melaminschmelze auf eine Temperatur abgekühlt wird, die 1 bis 50°C, bevorzugt 1 bis 30°C über dem vom jeweiligen NH₃-Druck abhängigen Schmelzpunkt des Melamins liegt.

Üblicherweise sind dies 1 bis 5 Gew.% der insgesamt zur Herstellung des Melamins nötigen Harnstoffmenge, bevorzugt 2 bis 3 Gew.%. Der Harnstoff kommt bevorzugt aus dem Offgaswäscher und hat demgemäß NH₃ gelöst. Es ist aber auch möglich, Harnstoffschmelze direkt aus der Harnstoffanlage praktisch ammoniakfrei, oder Harnstoff, gelöst in flüssigem NH₃, einzutragen.
Weiters wird mit dem Harnstoff eine geringe Menge an Wasser, entsprechend dem jeweiligen Wassergehalt von technischem Harnstoff eingebracht. Die eingebrachte Wassermenge beträgt 0,1 - 5 Gew.% Wasser, bevorzugt 0,1 - 3 Gew.%, bezogen auf zugeführten Harnstoff. Weiters wird frisches NH₃-Gas zugeführt, und es erfolgt hier aufgrund der endothermen Reaktion unter Temperaturerniedrigung der Schmelze die Umsetzung des restlichen Teils der Gesamtharnstoffmenge zu Melamin und Offgas, das wiederum hauptsächlich aus CO₂ und NH₃ sowie Spuren von gasförmigem Melamin besteht. Dabei kommt die für die Umsetzung des restlichen Harnstoffs zu Melamin nötige Wärmemenge aus der vom Melaminreaktor stammenden vorhandenen Melaminschmelze, die dabei gleichzeitig auf die gewünschte Temperatur abgekühlt wird.
Die Temperatur des zugeführten Harnstoffs liegt zwischen etwa 135°C und 250°C, bevorzugt zwischen etwa 170 und 220 °C, die Temperatur des gasförmigen NH₃ liegt zwischen etwa 150 und 450 °C, die Einbringung beider Stoffe in den Kühlreaktor erfolgt von unten in fein verteilter Form.
Dabei beträgt das Molverhältnis von dem Kühlreaktor zugeführtem NH₃ zur im Kühlreaktor vorhandenen Melaminmenge etwa 0,1-10mol, bevorzugt 0,1-5mol, besonders bevorzugt 0,1-2mol NH₃.

Der Druck im Kühlreaktor kann gleich, niedriger oder höher als der Druck im Melaminreaktor sein. Bevorzugt ist der Druck im Kühlreaktor etwa gleich wie im Melaminreaktor und liegt in einem Bereich von etwa 50 bis 350 bar, bevorzugt von etwa 80 bis 250 bar. Die Temperatur im Kühlreaktor ist niedriger als die Temperatur im Melaminreaktor und liegt meist in einem Bereich von etwa 300 bis 350 °C.
Die Temperatur ist im Kühlreaktor unter Berücksichtigung des druckabhängigen Melaminschmelzpunktes so zu wählen, daß das Melamin zu jeder Zeit, in der flüssigen Phase vorliegt und die im Reaktor herrschende Temperatur bevorzugt so nahe wie möglich beim jeweiligen Schmelzpunkt liegt.
Als Kühlreaktor kann ein beliebiger Reaktor verwendet werden, beispielsweise ein vertikaler, gegebenenfalls mit Packungselementen versehener Behälter, der beim Betrieb bevorzugt zu über 60 % mit der Melaminschmelze gefüllt ist, oder ein Rührreaktor.
Der Kühlreaktor kann auch als Fallfilmreaktor ausgebildet sein. In diesem Falle besteht er im wesentlichen aus einem oder mehreren Rohren, in denen die Melaminschmelze von oben nach unten fließt, während gasförmiges Ammoniak im Gegenstrom durch die Melaminschmelze bzw. über den Melaminschmelzefilm nach oben geleitet wird. Durch die gleichmäßige Benetzung der Rohre mit dem abfließenden Melaminstrom ergibt sich eine nahezu konstante Schichtdicke des Melaminfilmes an der Rohrinnenwand.

In einer weiteren Ausführungsform ist der Kühlreaktor ein Kombireaktor, dessen oberer Teil als Tankreaktor und dessen unterer Teil als Fallfilmreaktor ausgebildet ist. Dabei ist es vorteilhaft, die aus dem Fallfilmreaktor abgetrennten Gase in den Melaminreaktor rückzuführen. Weiters ist es vorteilhaft, die aus dem Fallfilmreaktor abgetrennten Gase in den Tankreaktor einzuleiten. Der Kühlreaktor kann auch aus mehreren übereinander angeordneten Compartments bestehen, die durch Böden, beispielsweise Ventilböden voneinander abgetrennt sind.

Im Kühlreaktor wird der in der aus dem Melaminreaktor kommenden Melaminschmelze enthaltene, noch nicht umgesetzte Harnstoff nahezu vollständig zu Melamin und Offgas umgesetzt. Gleichzeitig werden die in der Melaminschmelze enthaltenen Nebenprodukte wie z.B. Melem, Melam, Ammelin oder Ammelid im Kühlreaktor unter NH₃-Atmosphäre in Melamin umgewandelt.
Das hauptsächlich aus CO₂, NH₃ und geringen Mengen an gasförmigem Melamin bestehende Offgas wird kontinuierlich am Kopf des Kühlreaktors entfernt und entweder dem Offgaswäscher zugeführt oder bevorzugt in den Melaminreaktor rückgeführt. Durch die günstige Reaktionsführung im Kühlreaktor wird am Ausgang desselben eine Melaminreinheit von bis zu 99 % erreicht.

Falls eine höhere Reinheit im Endprodukt gewünscht wird, kann das im Kühlreaktor erhaltene Melamin mit oder ohne Druckerhöhung, mit weiterer NH₃-Zufuhr bei gleichzeitiger weiterer Temperaturerniedrigung durch einen Nachreaktor geleitet werden. Im Nachreaktor kann die Schmelzetemperatur weiter abgesenkt werden, ohne daß eine Verfestigung des Melamins erfolgt. Die Temperatur im Nachreaktor liegt dabei wieder 1 bis 50°C, bevorzugt 1 bis 30°C höher als der vom jeweils herrschenden NH₃-Druck abhängige Schmelzpunkt des Melamins. Der Druck im Nachreaktor kann bis zu 1000 bar erreichen, er liegt üblicherweise bei etwa 100 bis 500bar, bevorzugt bei 150 bis 350 bar.

Bevorzugt werden die Melaminschmelze und NH₃ von unten in den Nachreaktor eingeführt und am Kopf abgeführt. Der Nachreaktor besteht beispielsweise aus einer Kolonne mit Einbauten, die eine gleichmäßige Gasverteilung und Kühlung der Melaminschmelze gewährleisten. Diese Einbauten können beispielsweise Packungen, oder ein Statikmixer sein. Die Kühlung erfolgt durch das zugeführte kalte NH₃ oder geeignete Kühleinrichtungen.

Die anschließende Verfestigung des Melamins erfolgt auf beliebige Weise, beispielsweise durch Entspannen des mit Ammoniak gesättigten Melamins bei einer Temperatur, die knapp über ihrem druckabhängigen Schmelzpunkt liegt, durch Verfestigen in einer Wirbelschicht oder durch Quenchen mit Wasser, mit flüssigem oder gasförmigem Ammoniak oder durch Sublimieren und anschließendes Desublimieren aus der Gasphase.

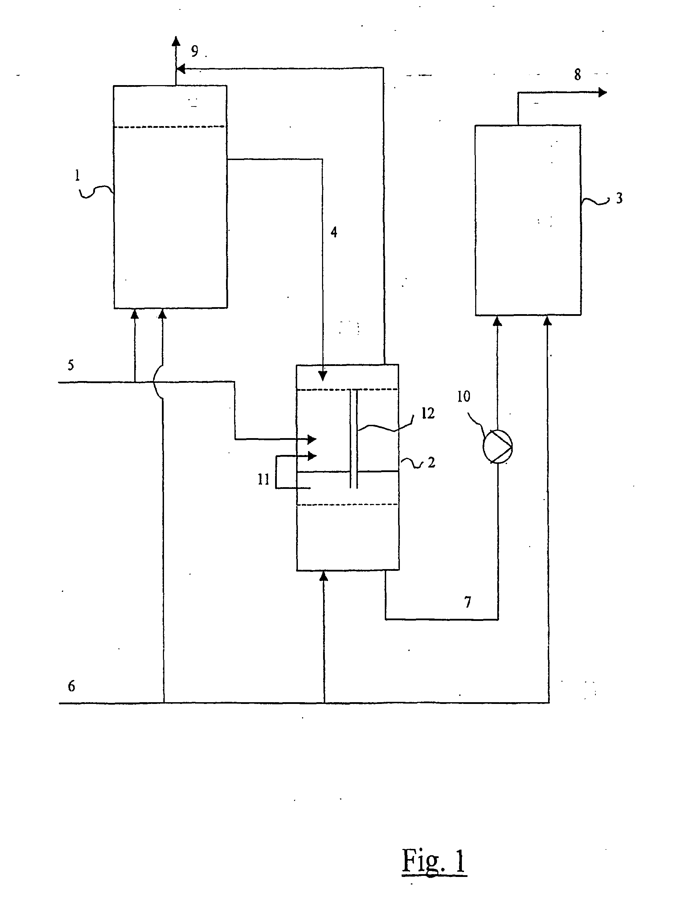

Fig. 1 stellt schematisch eine mögliche Anordnung zur Durchführung des erfindungsgemäßen Verfahrens dar. Es bedeuten (1) einen Melaminreaktor, (2) einen aus 2 Compartments bestehenden Kühlreaktor mit Überlaufrohr (12) für die Melaminschmelze, (3) einen Nachreaktor. (4) ist die aus dem Melaminreaktor in den Kühlreaktor strömende Melaminschmelze, (5) ist eine Harnstoffschmelze, die sowohl in den Melaminreaktor (1), als auch in den Kühlreaktor (2) eingebracht wird, (6) ist NH₃-Gas zur Einleitung in den Melaminreaktor (1), in den Kühlreaktor (2) und in den Nachreaktor (3). (7) ist die aus dem Kühlreaktor (2) über Pumpe(1 0) in den Nachreaktor (3) geführte Melaminschmelze, (8) die zur weiteren Aufarbeitung aus dem Nachreaktor (3) kommende Melaminschmelze. (9) sind die Offgase aus dem Melaminreaktor (1) und dem Kühlreaktor (2). (11) ist das Offgas aus dem unteren Compartment des Kühlreaktors (2), das entweder als Strippgas in das obere Compartment des Kühlreaktors (2) oder in den Melaminreaktor (1) rückgeführt wird.

### Beispiel:

In einen mit Sulzer-Packungen gefüllten Kühlreaktor, Höhe 4,5 m, Durchmesser 0,8 m, der einen Druck von 130 bar und eine Temperatur von 380°C aufweist, werden von oben 4103 kg/h Melaminschmelze und 370 kg/h Harnstoff eingebracht. Im Gegenstrom werden 1152 kg/h NH₃-Gas einer Temperatur von 350°C von unten durch den Kühlreaktor geleitet und die Gase am Kopf des Kühlreaktors abgezogen und dem Hauptreaktor zugeführt. Am Boden des Kühlreaktors werden 4395 kg/h der mit NH₃ gesättigten Melaminschmelze mit einer Reinheit von 99,0 % und einer Temperatur von 350°C abgezogen und gemeinsam mit 295 kg/h NH₃-Gas durch einen Nachreaktor, gefüllt mit statischen Mischelementen (Sulzer Mischerpackungen), der eine Höhe von 6 m, einen Durchmesser von 0,3 m aufweist und der bei einem Druck von 250 bar und einer Temperatur von 325°C betrieben wird, geleitet. Am Ausgang des Nachreaktors werden 4690 kg/h einer mit NH₃ gesättigten Melaminschmelze erhalten. Das erhaltene Melamin weist eine Reinheit von 99,6 % auf.

## Patentansprüche

1. Verfahren zur Herstellung von Melamin durch Pyrolyse von Harnstoff in einem Hochdruckverfahren, **dadurch gekennzeichnet, daß** Harnstoff gegebenenfalls gemeinsam mit NH₃ einem Melaminreaktor zugeführt, dort zu Melamin umgesetzt und das entstehende Offgas am Kopf des Reaktors abgezogen wird, die gebildete Melaminschmelze über einen Überlauf von oben einem Kühlreaktor zugeführt wird und im Kühlreaktor mit einer solchen Menge Harnstoffs versetzt wird, daß sie auf eine Temperatur abgekühlt wird, die 1 - 50 °C, bevorzugt 1 - 30 °C über dem vom jeweiligen NH₃-Druck abhängigen Schmelzpunkt des Melamins liegt, worauf durch Einleiten von NH₃ im Gegenstrom das gebildete CO₂ ausgetrieben, die Gase am Kopf des Kühlreaktors abgetrennt werden und die Melaminschmelze anschließend in beliebiger Weise aufgearbeitet wird.

2. Verfahren gemäß Anspruch 1, **dadurch gekennzeichnet, daß** 1 - 5 Gew.%, bevorzugt 2 - 3 Gew.% der insgesamt zur Herstellung des Melamins nötigen Harnstoffmenge in den Kühlreaktor eingebracht werden.

3. Verfahren gemäß Anspruch 1, **dadurch gekennzeichnet, daß** der in den Kühlreaktor eingetragene Harnstoff aus dem Offgaswäscher, und/oder aus der Harnstoffanlage kommt.

4. Verfahren gemäß Anspruch 1, **dadurch gekennzeichnet, daß** der in den Kühlreaktor eingetragene Harnstoff in flüssigem NH₃ gelöst ist.

5. Verfahren gemäß Anspruch 1, **dadurch gekennzeichnet, daß** der Harnstoff einen Wassergehalt von 0,1 - 5 Gew. %, bevorzugt von 0,1-3 Gew.%, aufweist.

6. Verfahren gemäß Anspruch 1, **dadurch gekennzeichnet, daß** der Kühlreaktor ein Tankreaktor ist.

7. Verfahren gemäß Anspruch 1, **dadurch gekennzeichnet, daß** der Kühlreaktor aus mehreren übereinander angeordneten Compartments besteht.

8. Verfahren gemäß Anspruch 1, **dadurch gekennzeichnet, daß** der Kühlreaktor ein Fallfilmreaktor ist.

9. Verfahren gemäß Anspruch 1, **dadurch gekennzeichnet, daß** der Kühlreaktor ein Kombireaktor ist, dessen oberer Teil als Tankreaktor und dessen unterer Teil als Fallfilmreaktor ausgebildet ist.

10. Verfahren gemäß Anspruch 6, **dadurch gekennzeichnet, daß** die aus dem Fallfilmreaktor abgetrennten Gase in den Tankreaktor geleitet werden.

11. Verfahren gemäß Anspruch 1, **dadurch gekennzeichnet, daß** die aus dem Kühlreaktor abgetrennten Gase in den Melaminreaktor zurückgeführt werden.

12. Verfahren gemäß Anspruch 1, **dadurch gekennzeichnet, daß** das im Kühlreaktor gebildete Melamin gegebenenfalls unter Druckerhöhung auf 100 bar bis 1000 bar und Temperaturerniedrigung auf einen Wert, der 1 bis 50°C, bevorzugt 1 bis 30°C über dem vom jeweiligen NH₃-Druck abhängigen Schmelzpunkt des Melamins liegt, einem Nachreaktor zugeführt wird und anschließend in beliebiger Weise aufgearbeitet wird.

## Claims

1. Process for preparing melamine by pyrolysis of urea in a high-pressure process, **characterized in that** urea is, optionally together with NH₃, fed to a melamine reactor, there converted into melamine and the resulting offgas is taken off at the top of the reactor, the melamine melt formed is fed from the top via an overflow into a cooling reactor and admixed in the cooling reactor with such an amount of urea that it is cooled to a temperature which is 1 - 50°C, preferably 1 - 30°C, above the melting point of the melamine, which is dependent on the prevailing NH₃ pressure, after which the CO₂ formed is driven out by introduction of NH₃ in countercurrent, the gases are separated off at the top of the cooling reactor and the melamine melt is subsequently worked up in any appropriate way.

2. Process according to Claim 1, **characterized in that** 1 - 5% by weight, preferably 2 - 3% by weight, of the total amount of urea required for preparing the melamine are introduced into the cooling reactor.

3. Process according to Claim 1, **characterized in that** the urea introduced into the cooling reactor comes from the offgas scrubber and/or from the urea plant.

4. Process according to Claim 1, **characterized in that** the urea introduced into the cooling reactor is in the form of a solution in liquid NH₃.

5. Process according to Claim 1, **characterized in that** the urea has a water content of 0.1 - 5% by weight, preferably 0.1 - 3% by weight.

6. Process according to Claim 1, **characterized in that** the cooling reactor is a tank reactor.

7. Process according to Claim 1, **characterized in that** the cooling reactor is a falling film reactor.

8. Process according to Claim 1, **characterized in that** the cooling reactor comprises a plurality of compartments arranged one above the other.

9. Process according to Claim 1, **characterized in that** the cooling reactor is a combination reactor whose upper part is configured as a tank reactor and whose lower part is configured as a falling film reactor.

10. Process according to Claim 6, **characterized in that** the gases separated off from the falling film reactor are conveyed into the tank reactor.

11. Process according to Claim 1, **characterized in that** the gases separated off from the cooling reactor are recirculated to the melamine reactor.

12. Process according to Claim 1, **characterized in that** the melamine formed in the cooling reactor is, optionally with an increase in pressure to from 100 bar to 1000 bar and a decrease in temperature to a value which is from 1 to 50°C, preferably from 1 to 30°C, above the melting point of the melamine which is dependent on the prevailing NH₃ pressure, fed to an after-reactor and is subsequently worked up in any appropriate way.

## Revendications

1. Procédé pour la préparation de mélamine par pyrolyse d'urée dans un procédé haute pression, **caractérisé en ce que** l'urée est alimentée, le cas échéant simultanément avec NH₃, dans un réacteur à mélamine, y est transformée en mélamine et les effluents gazeux formés sont évacués en tête du réacteur, la masse fondue de mélamine formée est introduite via une surverse à partir du haut dans un réacteur de refroidissement et est mélangée dans le réacteur de refroidissement avec une quantité d'urée telle qu'elle est refroidie à une température qui est supérieure de 1-50°C, de préférence de 1-30°C au point de fusion de la mélamine dépendant de la pression respective en NH₃, suite à quoi, par l'introduction de NH₃ à contre-courant, le CO₂ formé est évacué, les gaz en tête du réacteur de refroidissement sont séparés et la masse fondue de mélamine est ensuite traitée de manière quelconque.

2. Procédé selon la revendication 1, **caractérisé en ce qu'**on introduit 1-5% en poids, de préférence 2-3% en poids de la quantité d'urée totale nécessaire pour la préparation de la mélamine dans le réacteur de refroidissement.

3. Procédé selon la revendication 1, **caractérisé en ce que** l'urée introduite dans le réacteur de refroidissement provient du laveur des effluents gazeux et/ou de l'installation d'urée.

4. Procédé selon la revendication 1, **caractérisé en ce que** l'urée introduite dans le réacteur de refroidissement est dissoute dans du NH₃ liquide.

5. Procédé selon la revendication 1, **caractérisé en ce que** l'urée présente une teneur en eau de 0,1-5% en poids, de préférence de 0,1-3% en poids.

6. Procédé selon la revendication 1, **caractérisé en ce que** le réacteur de refroidissement est un réacteur-cuve.

7. Procédé selon la revendication 1, **caractérisé en ce que** le réacteur de refroidissement est un réacteur à film tombant.

8. Procédé selon la revendication 1, **caractérisé en ce que** le réacteur de refroidissement est constitué par plusieurs compartiments disposés les uns au-dessus des autres.

9. Procédé selon la revendication 1, **caractérisé en ce que** le réacteur de refroidissement est un réacteur combiné dont la partie supérieure est réalisée sous forme de réacteur-cuve et la partie inférieure sous forme de réacteur à film tombant.

10. Procédé selon la revendication 6, **caractérisé en ce que** les gaz séparés du réacteur à film tombant sont guidés dans le réacteur-cuve.

11. Procédé selon la revendication 1, **caractérisé en ce que** les gaz séparés du réacteur de refroidissement sont recyclés dans le réacteur à mélamine.

12. Procédé selon la revendication 1, **caractérisé en ce que** la mélamine formée dans le réacteur de refroidissement est alimentée, le cas échéant sous une augmentation de pression à 100 bars jusqu'à 1000 bars et une diminution de la température à une valeur qui est supérieure de 1 à 50°C, de préférence de 1 à 30°C au point de fusion de la mélamine dépendant de la pression respective en NH₃, dans un post-réacteur et est ensuite traitée de manière quelconque.
